# EUROPEAN PATENT SPECIFICATION

(11) **EP 2 978 439 B1**
(45) Date of publication and mention of the grant of the patent: **16.08.2017**
(21) Application number: 14717212.6
(22) Date of filing: 19.03.2014
(51) Int. Cl.: A61K 38/10, A61K 31/513, A61P 35/02

(54) **A COMBINATION OF A CXCR4 ANTAGONIST AND CYTARABINE FOR USE IN TREATING MYELOID LEUKEMIA**
EINE KOMBINATION VON EINEM CXCR4 ANTAGONIST UND CYTARABINE ZUR VERWENDUNG IN DER BEHANDLUNG VON MYELOISCHER LEUKÄMIE
UNE COMBINAISON D'UN ANTAGONISTE CXCR4 ET CYTARABINE POUR L'UTILISATION DANS LE TRAITEMENT DE LA LEUCÉMIE MYÉLOÏDE

(30) Priority: 24.03.2013 US 201361804677 P
(43) Date of publication of application: 03.02.2016
(73) Proprietor: Biokine Therapeutics Ltd., 7403617 Nes Ziona (IL); BIOLINERX LTD., 7177871 Modiln (IL)
(72) Inventor: PELED, Amnon, 6777634 Tel-Aviv (IL); PEREG, Yaron, 6085000 Shoham (IL)
(74) Representative: Jacobi, Markus Alexander
(86) International application number: PCT/IL2014/050303
(87) International publication number: WO 2014/155376

(56) References cited:
- UY G L ET AL: "A phase 1/2 study of chemosensitization with the CXCR4 antagonist plerixafor in relapsed or refractory acute myeloid leukemia.", BLOOD, vol. 119, 26 April 2012 (2012-04-26), pages 3917-3924, XP002725214,
- KATIA BEIDER ET AL: "CXCR4 antagonist 4F-benzoyl-TN14003 inhibits leukemia and multiple myeloma tumor growth", EXPERIMENTAL HEMATOLOGY, ELSEVIER INC, US, vol. 39, no. 3, 30 November 2010 (2010-11-30), pages 282-292, XP028154790, ISSN: 0301-472X, DOI: 10.1016/J.EXPHEM.2010.11.010 [retrieved on 2010-12-05]
- MANDAWAT A: "Pan-histone deacetylase inhibitor panobinostat depletes CXCR4 levels and signaling and exerts synergistic antimyeloid activity in combiantion with CXCR4 antagonist", BLOOD, vol. 116, no. 24, 9 December 2010 (2010-12-09), pages 5306-5315, XP002725236,
- QIN T ET AL: "Effect of Cytarabine and Decitabine in combination in human leukemic cell lines", CLIN CANCER RES, vol. 13, no. 14, 15 July 2007 (2007-07-15) , XP002725241,

### SEQUENCE LISTING

<110> BIOKINE THERAPEUTICS LTD.
   BIOLINERX LTD.
   PELED, Amnon
   PEREG, Yaron
<120> METHODS OF TREATING MYELOID LEUKEMIA
<130> 58373
<150> US 61/804,677
   <151> 2013-03-24
<160> 72
<170> PatentIn version 3.5
<210> 1
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' AMIDATED
<400> 1
   Xaa Arg Xaa Cys Tyr Xaa Lys Xaa Pro Tyr Arg Xaa Cys Arg
   1               5                   10
<210> 2
   <211> 14
   <212> PRT
   <213> Artificial
<220>
   <223> SYNTHETIC PEPTIDE
<220>
   <221> MOD_RES
   <222> (1)..(1)
   <223> N' ACETYLATION
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<400> 2
   Arg Arg Xaa Cys Tyr Xaa Lys Xaa Pro Tyr Arg Xaa Cys Arg
   1               5                   10
<210> 3
   <211> 14
   <212> PRT
   <213> Artificial
<220>
   <223> SYNTHETIC PEPTIDE
<220>
   <221> MOD_RES
   <222> (1)..(1)
   <223> N' ACETYLATION
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<400> 3
   Arg Arg Xaa Cys Tyr Arg Lys Xaa Pro Tyr Arg Xaa Cys Arg
   1               5                   10
<210> 4
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<400> 4
   Arg Arg Xaa Cys Tyr Xaa Lys Xaa Pro Tyr Arg Xaa Cys Arg
   1               5                   10
<210> 5
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (11)..(12)
   <223> citrulline
<400> 5
   Arg Arg Xaa Cys Tyr Xaa Lys Xaa Pro Tyr Xaa Xaa Cys Arg
   1               5                   10
<210> 6
   <211> 14
   <212> PRT
   <213> Artificial
<220>
   <223> synthetic peptide
<220>
   <221> MOD_RES
   <222> (1)..(1)
   <223> N' acetylated citrulline
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<400> 6
   Xaa Arg Xaa Cys Tyr Xaa Lys Xaa Pro Tyr Arg Xaa Cys Arg
   1               5                   10
<210> 7
   <211> 14
   <212> PRT
   <213> Artificial
<220>
   <223> synthetic peptide
<220>
   <221> MOD_RES
   <222> (1)..(1)
   <223> N' acetylated citrulline
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<400> 7
   Xaa Arg Xaa Cys Tyr Arg Lys Xaa Pro Tyr Arg Xaa Cys Arg
   1               5                   10
<210> 8
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (11)..(12)
   <223> citrulline
<400> 8
   Arg Arg Xaa Cys Tyr Arg Lys Xaa Pro Tyr Xaa Xaa Cys Arg
   1               5                   10
<210> 9
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (11)..(12)
   <223> citrulline
<400> 9
   Xaa Arg Xaa Cys Tyr Arg Lys Xaa Pro Tyr Xaa Xaa Cys Arg
   1               5                   10
<210> 10
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 10
   Arg Arg Xaa Cys Tyr Xaa Lys Xaa Pro Tyr Arg Xaa Cys Arg
   1               5                   10
<210> 11
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (11)..(12)
   <223> citrulline
<220>
   <221> MOD_RES
   <222> (14)..(14)
   <223> C' AMIDATED
<400> 11
   Arg Arg Xaa Cys Tyr Xaa Lys Xaa Pro Tyr Xaa Xaa Cys Arg
   1               5                   10
<210> 12
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> AMIDATED
<400> 12
   Xaa Arg Xaa Cys Tyr Xaa Lys Xaa Pro Tyr Arg Xaa Cys Arg
   1               5                   10
<210> 13
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 13
   Xaa Arg Xaa Cys Tyr Arg Lys Xaa Pro Tyr Arg Xaa Cys Arg
   1               5                   10
<210> 14
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (11)..(12)
   <223> citrulline
<220>
   <221> MOD_RES
   <222> (14)..(14)
   <223> C' AMIDATED
<400> 14
   Arg Arg Xaa Cys Tyr Arg Lys Xaa Pro Tyr Xaa Xaa Cys Arg
   1               5                   10
<210> 15
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (11)..(12)
   <223> citrulline
<220>
   <221> MOD_RES
   <222> (14)..(14)
   <223> C' AMIDATED
<400> 15
   Xaa Arg Xaa Cys Tyr Arg Lys Xaa Pro Tyr Xaa Xaa Cys Arg
   1               5                   10
<210> 16
   <211> 14
   <212> PRT
   <213> Artificial
<220>
   <223> synthetic peptide
<220>
   <221> MOD_RES
   <222> (1)..(1)
   <223> D-glutamic acid
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 16
   Xaa Arg Xaa Cys Tyr Arg Lys Xaa Pro Tyr Arg Xaa Cys Arg
   1               5                   10
<210> 17
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 17
   Arg Glu Xaa Cys Tyr Arg Lys Xaa Pro Tyr Arg Xaa Cys Arg
   1               5                   10
<210> 18
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 18
   Arg Arg Xaa Cys Tyr Glu Lys Xaa Pro Tyr Arg Xaa Cys Arg
   1               5                   10
<210> 19
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 19
   Arg Arg Xaa Cys Tyr Arg Glu Xaa Pro Tyr Arg Xaa Cys Arg
   1               5                   10
<210> 20
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-glutamic acid
<220>
   <221> MOD_RES
   <222> (12)..(12)
   <223> citrulline
<400> 20
   Arg Arg Xaa Cys Tyr Arg Lys Xaa Pro Tyr Arg Xaa Cys Arg
   1               5                   10
<210> 21
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 21
   Arg Arg Xaa Cys Tyr Arg Lys Xaa Pro Tyr Glu Xaa Cys Arg
   1               5                   10
<210> 22
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 22
   Arg Arg Xaa Cys Tyr Arg Lys Xaa Pro Tyr Arg Xaa Cys Glu
   1               5                   10
<210> 23
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> C' amidated
<400> 23
   Arg Arg Xaa Cys Tyr Xaa Lys Xaa Pro Tyr Arg Xaa Cys Arg
   1               5                   10
<210> 24
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> D-glutamic acid
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> c' amidated
<400> 24
   Arg Arg Xaa Cys Tyr Xaa Lys Xaa Pro Tyr Arg Xaa Cys Arg
   1               5                   10
<210> 25
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> D-glutamic acid
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 25
   Arg Arg Xaa Cys Tyr Xaa Lys Xaa Pro Tyr Arg Xaa Cys Arg
   1               5                   10
<210> 26
   <211> 14
   <212> PRT
   <213> Artificial
<220>
   <223> synthetic peptide
<220>
   <221> MOD_RES
   <222> (1)..(1)
   <223> D-glutamic acid
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 26
   Xaa Arg Xaa Cys Tyr Xaa Lys Xaa Pro Tyr Arg Xaa Cys Arg
   1               5                   10
<210> 27
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (10)..(10)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 27
   Arg Arg Xaa Cys Tyr Xaa Lys Xaa Pro Xaa Arg Xaa Cys Arg
   1               5                   10
<210> 28
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> D-glutamic acid
<220>
   <221> MOD_RES
   <222> (14)..(14)
   <223> c' amidated
<400> 28
   Arg Arg Xaa Cys Tyr Xaa Lys Xaa Pro Tyr Arg Xaa Cys Arg
   1               5                   10
<210> 29
   <211> 14
   <212> PRT
   <213> Artificial
<220>
   <223> synthetic peptide
<220>
   <221> MOD_RES
   <222> (1)..(1)
   <223> N' ACETYLATED D-glutamic acid
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 29
   Xaa Arg Xaa Cys Tyr Xaa Lys Xaa Pro Tyr Arg Xaa Cys Arg
   1               5                   10
<210> 30
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (10)..(10)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 30
   Arg Arg Xaa Cys Tyr Xaa Lys Xaa Pro Xaa Arg Xaa Cys Arg
   1               5                   10
<210> 31
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> D-glutamic acid
<220>
   <221> MOD_RES
   <222> (14)..(14)
   <223> c' amidated
<400> 31
   Arg Arg Xaa Cys Tyr Xaa Lys Xaa Pro Tyr Arg Xaa Cys Arg
   1               5                   10
<210> 32
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 32
   Arg Arg Xaa Cys Tyr Xaa Lys Xaa Pro Tyr Arg Xaa Cys Arg
   1               5                   10
<210> 33
   <211> 14
   <212> PRT
   <213> Artificial
<220>
   <223> synthetic peptide
<220>
   <221> MOD_RES
   <222> (1)..(1)
   <223> Guanyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 33
   Xaa Arg Xaa Cys Tyr Xaa Lys Xaa Pro Tyr Arg Xaa Cys Arg
   1               5                   10
<210> 34
   <211> 14
   <212> PRT
   <213> Artificial
<220>
   <223> synthetic peptide
<220>
   <221> MOD_RES
   <222> (1)..(1)
   <223> Tetramethylguanyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 34
   Xaa Arg Xaa Cys Tyr Xaa Lys Xaa Pro Tyr Arg Xaa Cys Arg
   1               5                   10
<210> 35
   <211> 13
   <212> PRT
   <213> Artificial
<220>
   <223> synthetic peptide
<220>
   <221> MOD_RES
   <222> (1)..(1)
   <223> Tetramethylguanyl-arginine
<220>
   <221> MOD_RES
   <222> (2)..(2)
   <223> 3-((2-naphthyl) alanine
<220>
   <221> MOD_RES
   <222> (5)..(5)
   <223> citrulline
<220>
   <221> MOD_RES
   <222> (7).. (7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 35
   Xaa Xaa Cys Tyr Xaa Lys Xaa Pro Tyr Arg Xaa Cys Arg
   1               5                   10
<210> 36
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> amidated
<400> 36
   Xaa Arg Xaa Cys Tyr Xaa Lys Xaa Pro Tyr Arg Xaa Cys Arg
   1               5                   10
<210> 37
   <211> 14
   <212> PRT
   <213> Artificial
<220>
   <223> synthetic peptide
<220>
   <221> MOD_RES
   <222> (1)..(1)
   <223> 2-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 37
   Xaa Arg Xaa Cys Tyr Xaa Lys Xaa Pro Tyr Arg Xaa Cys Arg
   1               5                   10
<210> 38
   <211> 13
   <212> PRT
   <213> Artificial
<220>
   <223> synthetic peptide
<220>
   <221> MOD_RES
   <222> (1)..(1)
   <223> 5-aminopentanoyl-arginine
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7).. (7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 38
   Xaa Xaa Cys Tyr Xaa Lys Xaa Pro Tyr Arg Xaa Cys Arg
   1               5                   10
<210> 39
   <211> 14
   <212> PRT
   <213> Artificial
<220>
   <223> synthetic peptide
<220>
   <221> MOD_RES
   <222> (1)..(1)
   <223> 2-desamino-arginyl
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 39
   Xaa Arg Xaa Cys Tyr Xaa Lys Xaa Pro Tyr Arg Xaa Cys Arg
   1               5                   10
<210> 40
   <211> 13
   <212> PRT
   <213> Artificial
<220>
   <223> synthetic peptide
<220>
   <221> MOD_RES
   <222> (1)..(1)
   <223> Guanyl-arginine
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7)..(7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 40
   Xaa Xaa Cys Tyr Xaa Lys Xaa Pro Tyr Arg Xaa Cys Arg
   1               5                   10
<210> 41
   <211> 13
   <212> PRT
   <213> Artificial
<220>
   <223> synthetic peptide
<220>
   <221> MOD_RES
   <222> (1)..(1)
   <223> Succinyl-arginine
<220>
   <221> MOD_RES
   <222> (2)..(2)
   <223> 3-((2-naphthyl) alanine
<220>
   <221> MOD_RES
   <222> (5)..(5)
   <223> citrulline
<220>
   <221> MOD_RES
   <222> (7)..(7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 41
   Xaa Xaa Cys Tyr Xaa Lys Xaa Pro Tyr Arg Xaa Cys Arg
   1               5                   10
<210> 42
   <211> 13
   <212> PRT
   <213> Artificial
<220>
   <223> synthetic peptide
<220>
   <221> MOD_RES
   <222> (1)..(1)
   <223> Glutaryl-arginine
<220>
   <221> MOD_RES
   <222> (2)..(2)
   <223> 3-((2-naphthyl) alanine
<220>
   <221> MOD_RES
   <222> (5)..(5)
   <223> citrulline
<220>
   <221> MOD_RES
   <222> (7)..(7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 42
   Xaa Xaa Cys Tyr Xaa Lys Xaa Pro Tyr Arg Xaa Cys Arg
   1               5                   10
<210> 43
   <211> 14
   <212> PRT
   <213> Artificial
<220>
   <223> synthetic peptide
<220>
   <221> MISC_FEATURE
   <222> (1)..(1)
   <223> desaminoTMG-APA (formula IV in the specification)
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 43
   Xaa Arg Xaa Cys Tyr Xaa Lys Xaa Pro Tyr Arg Xaa Cys Arg
   1               5                   10
<210> 44
   <211> 14
   <212> PRT
   <213> Artificial
<220>
   <223> synthetic peptide
<220>
   <221> MISC_FEATURE
   <222> (1)..(1)
   <223> R-CH2 - formula (V) in the specification
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 44
   Xaa Arg Xaa Cys Tyr Xaa Lys Xaa Pro Tyr Arg Xaa Cys Arg
   1               5                   10
<210> 45
   <211> 13
   <212> PRT
   <213> Artificial
<220>
   <223> synthetic peptide
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7)..(7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 45
   Arg Xaa Cys Tyr Xaa Lys Xaa Pro Tyr Arg Xaa Cys Arg
   1               5                   10
<210> 46
   <211> 14
   <212> PRT
   <213> Artificial
<220>
   <223> synthetic peptide
<220>
   <221> MOD_RES
   <222> (1) . . (1)
   <223> tetramethylguanyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 46
   Xaa Arg Xaa Cys Tyr Xaa Lys Xaa Pro Tyr Arg Xaa Cys Arg
   1               5                   10
<210> 47
   <211> 14
   <212> PRT
   <213> Artificial
<220>
   <223> synthetic peptide
<220>
   <221> MOD_RES
   <222> (1) . . (1)
   <223> 6-aminohexanoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 47
   Xaa Arg Xaa Cys Tyr Xaa Lys Xaa Pro Tyr Arg Xaa Cys Arg
   1               5                   10
<210> 48
   <211> 14
   <212> PRT
   <213> Artificial
<220>
   <223> synthetic peptide
<220>
   <221> MOD_RES
   <222> (1) . . (1)
   <223> 6-aminohexanoyl-arginine
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 48
   Xaa Arg Xaa Cys Tyr Arg Lys Xaa Pro Tyr Arg Xaa Cys Arg
   1               5                   10
<210> 49
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' amidated
<400> 49
   Arg Arg Xaa Cys Tyr Xaa Arg Xaa Pro Tyr Arg Xaa Cys Arg
   1               5                   10
<210> 50
   <211> 14
   <212> PRT
   <213> Artificial
<220>
   <223> synthetic peptide
<220>
   <221> MOD_RES
   <222> (1) . . (1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' amidated
<400> 50
   Arg Arg Xaa Cys Tyr Xaa Arg Xaa Pro Tyr Arg Xaa Cys Arg
   1               5                   10
<210> 51
   <211> 14
   <212> PRT
   <213> Artificial
<220>
   <223> SYNTHETIC PEPTIDE
<220>
   <221> MOD_RES
   <222> (1) . . (1)
   <223> N' ACETYLATED
<220>
   <221> MOD_RES
   <222> (3)..(3)
   <223> 3-((2-naphthyl) alanine
<220>
   <221> MOD_RES
   <222> (6) . . (6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' AMIDATED
<400> 51
   Arg Arg Xaa Cys Tyr Xaa Lys Xaa Pro Tyr Arg Xaa Cys Arg
   1               5                   10
<210> 52
   <211> 14
   <212> PRT
   <213> Artificial
<220>
   <223> synthetic peptide
<220>
   <221> MOD_RES
   <222> (1) . . (1)
   <223> N' ACETYLATED
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 52
   Arg Arg Xaa Cys Tyr Arg Lys Xaa Pro Tyr Arg Xaa Cys Arg
   1               5                   10
<210> 53
   <211> 14
   <212> PRT
   <213> Artificial
<220>
   <223> synthetic peptide
<220>
   <221> MOD_RES
   <222> (1) . . (1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization by a NH-methyl group
<400> 53
   Xaa Arg Xaa Cys Tyr Xaa Lys Xaa Pro Tyr Arg Xaa Cys Arg
   1               5                   10
<210> 54
   <211> 14
   <212> PRT
   <213> Artificial
<220>
   <223> synthetic peptide
<220>
   <221> MOD_RES
   <222> (1) . . (1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization by a NH-ethyl group
<400> 54
   Xaa Arg Xaa Cys Tyr Xaa Lys Xaa Pro Tyr Arg Xaa Cys Arg
   1               5                   10
<210> 55
   <211> 14
   <212> PRT
   <213> Artificial
<220>
   <223> synthetic peptide
<220>
   <221> MOD_RES
   <222> (1) . . (1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization by NH-isopropyl
<400> 55
   Xaa Arg Xaa Cys Tyr Xaa Lys Xaa Pro Tyr Arg Xaa Cys Arg
   1               5                   10
<210> 56
   <211> 14
   <212> PRT
   <213> Artificial
<220>
   <223> synthetic peptide
<220>
   <221> MOD_RES
   <222> (1) . . (1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization with a tyramine residue
<400> 56
   Xaa Arg Xaa Cys Tyr Xaa Lys Xaa Pro Tyr Arg Xaa Cys Arg
   1               5                   10
<210> 57
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 57
   Ala Arg Xaa Cys Tyr Arg Lys Xaa Pro Tyr Arg Xaa Cys Arg
   1               5                   10
<210> 58
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 58
   Arg Arg Xaa Cys Tyr Ala Lys Xaa Pro Tyr Arg Xaa Cys Arg
   1               5                   10
<210> 59
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 59
   Arg Arg Xaa Cys Tyr Arg Ala Xaa Pro Tyr Arg Xaa Cys Arg
   1               5                   10
<210> 60
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-alanine
<220>
   <221> MOD_RES
   <222> (12)..(12)
   <223> citrulline
<400> 60
   Arg Arg Xaa Cys Tyr Arg Lys Xaa Pro Tyr Arg Xaa Cys Arg
   1               5                   10
<210> 61
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 61
   Arg Arg Xaa Cys Tyr Arg Lys Xaa Ala Tyr Arg Xaa Cys Arg
   1               5                   10
<210> 62
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 62
   Arg Arg Xaa Cys Tyr Arg Lys Xaa Pro Ala Arg Xaa Cys Arg
   1               5                   10
<210> 63
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 63
   Arg Arg Xaa Cys Tyr Arg Lys Xaa Pro Tyr Ala Xaa Cys Arg
   1 5 10
<210> 64
   <211> 14
   <212> PRT
   <213> Artificial
<220>
   <223> synthetic peptide
<220>
   <221> MOD_RES
   <222> (1) . . (1)
   <223> citrulline
<220>
   <221> MOD_RES
   <222> (3)..(3)
   <223> 3-((2-naphthyl) alanine
<220>
   <221> MOD_RES
   <222> (8) . . (8)
   <223> D-lysine
<220>
   <221> MOD_RES
   <222> (12)..(12)
   <223> citrulline
<400> 64
   Xaa Arg Xaa Cys Tyr Arg Lys Xaa Pro Tyr Arg Xaa Cys Arg
   1 5 10
<210> 65
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<400> 65
   Arg Arg Xaa Cys Tyr Xaa Lys Xaa Pro Tyr Arg Xaa Cys Arg
   1               5                   10
<210> 66
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' AMIDATED
<400> 66
   Arg Arg Xaa Cys Tyr Xaa Lys Xaa Pro Tyr Arg Xaa Cys Arg
   1               5                   10
<210> 67
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (7).. (7)
   <223> citrulline
<220>
   <221> MOD_RES
   <222> (8)..(8)
   <223> D-lysine
<220>
   <221> MOD_RES
   <222> (12)..(12)
   <223> citrulline
<400> 67
   Arg Arg Xaa Cys Tyr Arg Xaa Xaa Pro Tyr Arg Xaa Cys Arg
   1               5                   10
<210> 68
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8) . . (8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 68
   Arg Arg Xaa Cys Tyr Xaa Lys Xaa Pro Tyr Arg Xaa Cys Arg
   1               5                   10
<210> 69
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 69
   Arg Arg Xaa Cys Tyr Arg Lys Xaa Pro Tyr Arg Xaa Cys Arg
   1               5                   10
<210> 70
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6).. (6)
   <223> citrulline
<220>
   <221> MOD_RES
   <222> (8)..(8)
   <223> D-citrulline
<220>
   <221> MOD_RES
   <222> (12)..(12)
   <223> citrulline
<400> 70
   Arg Arg Xaa Cys Tyr Xaa Lys Xaa Pro Tyr Arg Xaa Cys Arg
   1               5                   10
<210> 71
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<400> 71
   Arg Arg Xaa Cys Tyr Arg Lys Xaa Pro Tyr Arg Xaa Cys Arg
   1               5                   10
<210> 72
   <211> 14
   <212> PRT
   <213> Artificial
<220>
   <223> synthetic peptide
<220>
   <221> MOD_RES
   <222> (1) . . (1)
   <223> citrulline
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' Amidated
<400> 72
   Xaa Arg Xaa Cys Tyr Arg Lys Xaa Pro Tyr Arg Xaa Cys Arg
   1               5                   10

## Description

### FIELD AND BACKGROUND OF THE INVENTION

The present invention relates to methods of treating myeloid leukemia and more particularly, to the use of a CXCR4-antagonistic peptide and a chemotherapeutic agent in the treatment of myeloid leukemia.

Acute myeloid leukemia (AML) is a heterogeneous group of diseases characterized by the uncontrolled proliferation of hematopoietic stem cells and progenitors (blasts) with a reduced capacity to differentiate into mature cells (Estey et al., Lancet 368:1894-1907, 2006). Despite sensitivity to chemotherapeutic, long-term disease-free survival for AML patients remains low and the majority eventually relapse from minimal residual disease (MRD; Matsunaga et al., Nat Med. 9:1158-65, 2003). Bone marrow (BM) is the major site for MRD where adhesion of AML cells to bone marrow components may provide protection from the drugs (Estey et al., Lancet 368:1894-1907, 2006). The chemokine receptor CXCR4 and its ligand stromal derived factor-1 (SDF-1/CXCL12) are important players involved in the cross-talk between leukemia cells and the BM microenvironment (J. A. Burger and A. Peled, Leukemia 23:43-52, 2009).

The bicyclam drug termed AMD3100, originally discovered as an anti-HIV compound, specifically interacts with CXCR4 in an antagonistic manner. Blocking CXCR4 receptor with AMD3100 results in the mobilization of hematopoietic progenitor cells. WO 2007/022523 discloses the use of CXCR4 agonists such as AMD3100 for enhancing the effectiveness of chemotherapeutic methods in subjects afflicted with myeloid or hematopoietic malignancies.

T-140 is a 14-residue synthetic peptide developed as a specific CXCR4 antagonist that suppress HIV-1 (X4-HIV-1) entry to T cells through specific binding to CXCR4 (Tamamura et al., Biochem. Biophys. Res. Commun. 253(3): 877-882, 1998). Subsequently, peptide analogs of T-140 were developed as specific CXCR4-antagonisic peptides with inhibitory activity at nanomolar levels [Tamamura et al. (Org. Biomol. Chem. 1: 3663-3669, 2003), WO 2002/020561, WO 2004/020462, WO 2004/087068, WO 00/09152, US 2002/0156034, and WO 2004/024178].

WO 2004/087068 discloses antagonists of chemokine receptors, particularly the CXCR4 receptor, and methods of their use, for example, in the treatment, prevention or diagnosis of cancer. The '068 publication discloses that exemplary CXCR4 peptide antagonists include T140 and derivatives of T140, and that the pathology includes cancer such as breast, brain, pancreatic, ovarian, prostate, kidney, and non-small lung cancer.

WO 00/09152 discloses a variety of therapeutic uses for CXCR4 antagonists such as in the treatment of cancer.

WO 2004/024178 discloses the use of a chemokine receptor antagonist as a ligand for the CXCR4 receptor for the apoptosis-inducing treatment and/or the prevention of the metastatic spread of cancer cells in a patient.

U.S. Publication No. 2002/0156034 discloses the use of CXCR4 antagonists for the treatment of hematopoietic cells such as in cancer.

WO 2002/020561 discloses peptide analogs and derivatives of T-140. The 561 publication demonstrates that the claimed peptides are potent CXCR4 inhibitors, manifesting high anti-HIV virus activity and low cytotoxicity.

Recently, a comparative study between the CXCR4 antagonists TN140 and AMD3100 suggested that TN140 is more effective than AMD3100 as a monotherapy in AML. TN140 and to a lesser extend AMD3100 induced regression of human CXCR4-expressing AML cells and targeted the NOD/Shi-scid/IL-2Rγnull (NOG) leukemia-initiating cells (LICs) (Y. Zhang et al., Cell Death and Disease, 2012).

WO 2004/020462 discloses additional novel peptide analogs and derivatives of T-140, including 4F-benzoyl-TN14003. The '462 publication further discloses preventive and therapeutic compositions and methods of using same utilizing T-140 analogs for the treatment of cancer, such as T-Cell leukemia.

Beider et al. (Exp. Hematol. 39:282-92, 2011) reported that 4F-benzoyl-TN14003 exhibits a CXCR4-dependent preferential cytotoxicity toward malignant cells of hematopoietic origin including AML. In vivo, subcutaneous injections of 4F-benzoyl-TN14003 significantly reduced the growth of human AML xenografts.

There it would be highly advantageous to have a safe and effective method for the treatment of myeloid leukemia.

### SUMMARY OF THE INVENTION

According to one aspect of the present invention there is provided a treatment myeloid leukemia. The treatment includes a step of administering to a subject in need thereof a therapeutically effective amount of a CXCR4-antagonistic peptide and a therapeutically effective amount of a chemotherapeutic agent as defined in claim 1. According to further features in preferred embodiments of the invention described below, the myeloid leukemia is acute myeloid leukemia.

The CXCR4-antagonistic peptide is as set forth in SEQ ID NO: 1 and the chemotherapeutic agent is cytarabine.

According to still further features in the described preferred embodiments the CXCR4-antagonistic peptide is administered to the subject in a daily amount between 0.1 to 10 mg per kg of body weight.

According to still further features in the described preferred embodiments cytarabine is administered to the subject in a daily amount between 1 to 10 g per square meter of body area.

According to still further features in the described preferred embodiments the CXCR4-antagonistic peptide is administered subcutaneously.

According to still further features in the described preferred embodiments cytarabine is administered intravenously.

According to still further features in the described preferred embodiments the CXCR4-antagonistic peptide is administered to the subject at least one day prior to the administration of the chemotherapeutic agent.

According to still further features in the described preferred embodiments the CXCR4-antagonistic peptide is administered to the subject at least one hour prior to the administration of said chemotherapeutic agent.

The present invention successfully addresses the shortcomings of the presently known configurations by providing a novel method of treating myeloid leukemia that is safe and effective.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

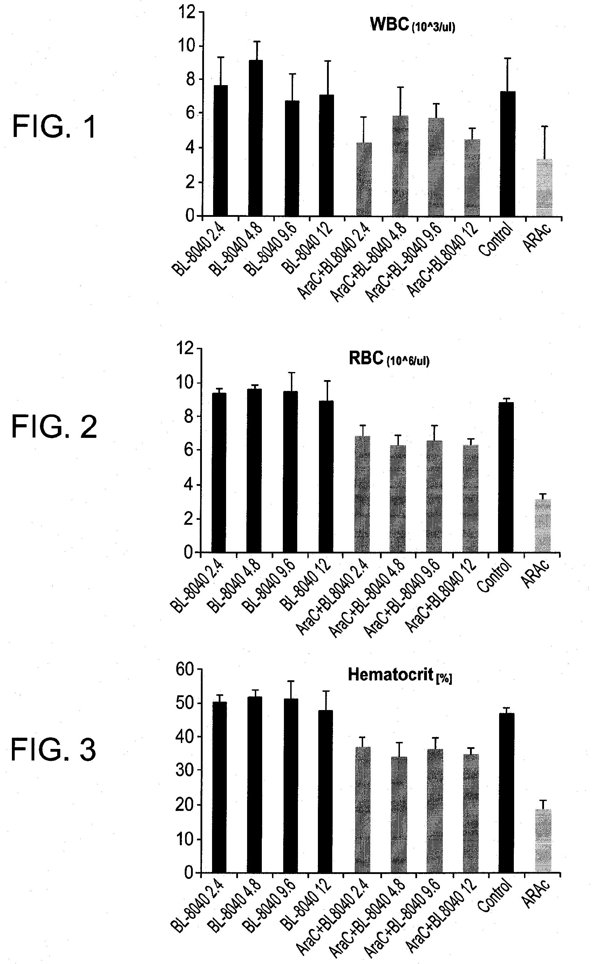

FIGs. 1-7 are bar graphs illustrating the effect of treating normal C57BL/6 mice with 4F-benzoyl-TN14003 (SEQ ID NO: 1, also referred to herein as BL-8040; at concentrations of 2.4, 4.8, 9.6, or 12 mg/Kg), cytarabine (ARA-C; at concentration of 200 mg/Kg), or combinations thereof, on blood counts performed five days after treatment.

Specifically:
FIG.1 illustrates the effect of BL-8040 alone, ARA-C, or a combination thereof, on the density of white blood cells (WBC; 10³/µl).
FIG. 2 illustrates the effect of BL-8040 alone, ARA-C, or a combination thereof, on the density of red blood cells (RBC; 10⁶/µl).
FIG. 3 illustrates the effect of BL-8040 alone, ARA-C, or a combination thereof, on the volume percentage of red blood cells in blood (Hematocrit; %).
FIG. 4 illustrates the effect of BL-8040 alone, ARA-C, or a combination thereof, on the density of hemoglobin (HGB; g / dl).
FIG. 5 illustrates the effect of BL-8040 alone, ARA-C, or a combination thereof, on the density of platelets density (10³/µl).
FIG. 6 illustrates the effect of BL-8040 alone, ARA-C, or a combination thereof, on the density of lymphocyte Abs (10³/µl).
FIG. 7 illustrates the effect BL-8040 alone, ARA-C, or a combination thereof, on the density of neutrophil Abs (10³/µl).

### DESCRIPTION OF SPECIFIC EMBODIMENTS OF THE INVENTION

The present invention, in some embodiments thereof, relates to uses of CXCR4-antagonistic peptides in combination with chemotherapeutic agents as claimed in claims 1-3 in the treatment of myeloid leukemia.

The principles and operation of the present invention may be better understood with reference to the drawings and accompanying descriptions.

Before explaining at least one embodiment of the invention in detail, it is to be understood that the invention is not necessarily limited in its application to the details exemplified by the Examples.

While reducing the present invention to practice, the present inventors have surprisingly uncovered that treating mice with the CXCR4-antagonistic peptide 4F-benzoyl-TN14003 (SEQ ID NO: 1), combined with the chemotherapeutic agent cytarabine (used in the treatment of myeloid leukemia), resulted in substantially higher levels of red blood-cells, hemoglobin and hematocrit, as compared to mice treated with the chemotherapeutic agent only (see Example 1). These results indicate that the CXCR4-antagonistic peptide is uniquely capable of alleviating non-target toxicity caused by the chemotherapeutic agent and therefore improves the safety as well as efficacy of myeloid leukemia chemotherapeutic treatment.

Thus, according to an aspect of the invention there is provided a treatment of myeloid leukemia in a subject. The treatment comprises administering to the subject a therapeutically effective amount of a CXCR4-antagonistic peptide and a therapeutically effective amount of a chemotherapeutic agent, as defined in the claims, thereby treating the myeloid leukemia in the subject.

As used herein a "CXCR4-antagonistic peptide" is a peptide which reduces CXCR-4 activation, by at least 10 %, as compared to same in the absence of the peptide antagonist. According to a specific instance the peptide antagonist is a competitive inhibitor. According to a specific instance the peptide antagonist is a non-competitive inhibitor.

As used herein, the term "peptide" encompasses native peptides (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body or more capable of penetrating into cells.

According to a specific instance, the peptide is no more than 100 amino acids in length. According to a specific instance, the peptide is 5-100 amino acids in length. According to a specific instance, the peptide is 5-50 amino acids in length. According to a specific instance, the peptide is 5-20 amino acids in length. According to a specific instance, the peptide is 5-15 amino acids in length. According to a specific embodiment, the peptide is 10-20 amino acids in length. According to a specific embodiment, the peptide is 10-15 amino acids in length.

According to specific instances, the CXCR4-antagonistic peptides of the present invention are for example , 4F-benzoyl-TN14003 (SEQ ID NO: 1). In various particular instances, the T-140 analog or derivative has an amino acid sequence as set forth in the following formula (I) or a salt thereof: wherein:
**A₁** is an arginine, lysine, ornithine, citrulline, alanine or glutamic acid residue or a N-α-substituted derivative of these amino acids, or A₁ is absent;
**A₂** represents an arginine or glutamic acid residue if A₁ is present, or A₂ represents an arginine or glutamic acid residue or a N-α-substituted derivative of these amino acids if A₁ is absent;
**A₃** represents an aromatic amino acid residue;
**A₄**, **A₅** and **A₉** each independently represents an arginine, lysine, ornithine, citrulline, alanine or glutamic acid residue;
**A₆** represents a proline, glycine, ornithine, lysine, alanine, citrulline, arginine or glutamic acid residue;
**A₇** represents a proline, glycine, ornithine, lysine, alanine, citrulline or arginine residue;
**A₈** represents a tyrosine, phenylalanine, alanine, naphthylalanine, citrulline or glutamic acid residue;
**A₁₀** represents a citrulline, glutamic acid, arginine or lysine residue;
**A₁₁** represents an arginine, glutamic acid, lysine or citrulline residue wherein the C-terminal carboxyl may be derivatized;
and the cysteine residue of the 4-position or the 13-position can form a disulfide bond, and the amino acids can be of either L or D form.

Exemplary peptides according to formula (I) are peptides having an amino acid sequence as set forth in any one of SEQ ID NOS:1-72, as presented in Table 1 hereinbelow.

**Table 1- T-140 and currently Preferred T-140 analogs**

| Analog | SEQ ID NO: | Amino acid sequence |
|---|---|---|
| 4F-benzoyl-TN14003 | 1 | 4F-benzoyl-Arg-Arg-Nal-Cys-Tyr-Cit-Lys-DLys-Pro-Tyr-Arg-Cit-Cys-Arg-NH₂ |
| AcTC14003 | 2 | Ac-Arg-Arg-Nal-Cys-Tyr-Cit-Lys-DLys-Pro-Tyr-Arg-Cit-Cys-Arg-OH |
| AcTC14005 | 3 | Ac-Arg-Arg-Nal-Cys-Tyr-Arg-Lys-DCit-Pro-Tyr-Arg-Cit-Cys-Arg-OH |
| AcTC14011 | 4 | Ac-Arg-Arg-Nal-Cys-Tyr-Cit-Lys-DCit-Pro-Tyr-Arg-Cit-Cys-Arg-OH |
| AcTC14013 | 5 | Ac-Arg-Arg-Nal-Cys-Tyr-Cit-Lys-DLys-Pro-Tyr-Cit-Cit-Cys-Arg-OH |
| AcTC14015 | 6 | Ac-Cit-Arg-Nal-Cys-Tyr-Cit-Lys-DLys-Pro-Tyr-Arg-Cit-Cys-Arg-OH |
| AcTC14017 | 7 | Ac-Cit-Arg-Nal-Cys-Tyr-Arg-Lys-DCit-Pro-Tyr-Arg-Cit-Cys-Arg-OH |
| AcTC14019 | 8 | Ac-Arg-Arg-Nal-Cys-Tyr-Arg-Lys-DCit-Pro-Tyr-Cit-Cit-Cys-Arg-OH |
| AcTC14021 | 9 | Ac-Cit-Arg-Nal-Cys-Tyr-Arg-Lys-DLys-Pro-Tyr-Cit-Cit-Cys-Arg-OH |
| AcTC14012 | 10 | Ac-Arg-Arg-Nal-Cys-Tyr-Cit-Lys-DCit-Pro-Tyr-Arg-Cit-Cys-Arg-NH₂ |
| AcTC14014 | 11 | Ac-Arg-Arg-Nal-Cys-Tyr-Cit-Lys-DLys-Pro-Tyr-Cit-Cit-Cys-Arg-NH₂ |
| AcTC14016 | 12 | Ac-Cit-Arg-Nal-Cys-Tyr-Cit-Lys-DLys-Pro-Tyr-Arg-Cit-Cys-Arg-NH₂ |
| AcTC14018 | 13 | Ac-Cit-Arg-Nal-Cys-Tyr-Arg-Lys-DCit-Pro-Tyr-Arg-Cit-Cys-Arg-NH₂ |
| AcTC14020 | 14 | Ac-Arg-Arg-Nal-Cys-Tyr-Arg-Lys-DCit-Pro-Tyr-Cit-Cit-Cys-Arg-NH₂ |
| AcTC14022 | 15 | Ac-Cit-Arg-Nal-Cys-Tyr-Arg-Lys-DLys-Pro-Tyr-Cit-Cit-Cys-Arg-NH₂ |
| TE14001 | 16 | H-DGlu-Arg-Nal-Cys-Tyr-Arg-Lys-DLys-Pro-Tyr-Arg-Cit-Cys-Arg-OH |
| TE14002 | 17 | H-Arg-Glu-Nal-Cys-Tyr-Arg-Lys-DLys-Pro-Tyr-Arg-Cit-Cys-Arg-OH |
| TE14003 | 18 | H-Arg-Arg-Nal-Cys-Tyr-Glu-Lys-DLys-Pro-Tyr-Arg-Cit-Cys-Arg-OH |
| TE14004 | 19 | H-Arg-Arg-Nal-Cys-Tyr-Arg-Glu-DLys-Pro-Tyr-Arg-Cit-Cys-Arg-OH |
| TE14005 | 20 | H-Arg-Arg-Nal-Cys-Tyr-Arg-Lys-DGlu-Pro-Tyr-Arg-Cit-Cys-Arg-OH |
| TE14006 | 21 | H-Arg-Arg-Nal-Cys-Tyr-Arg-Lys-DLys-Pro-Tyr-Glu-Cit-Cys-Arg-OH |
| TE14007 | 22 | H-Arg-Arg-Nal-Cys-Tyr-Arg-Lys-DLys-Pro-Tyr-Arg-Cit-Cys-Glu-OH |
| TE14011 | 23 | H-Arg-Arg-Nal-Cys-Tyr-Cit-Lys-DGlu-Pro-Tyr-Arg-Cit-Cys-Arg-NH₂ |
| TE14012 | 24 | H-Arg-Arg-Nal-Cys-Tyr-DGlu-Lys-DCit-Pro-Tyr-Arg-Cit-Cys-Arg-NH₂ |
| TE14013 | 25 | H-Arg-Arg-Nal-Cys-Tyr-DGlu-Lys-DGlu-Pro-Tyr-Arg-Cit-Cys-Arg-NH₂ |
| TE14014 | 26 | H-DGlu-Arg-Nal-Cys-Tyr-Cit-Lys-DGlu-Pro-Tyr-Arg-Cit-Cys-Arg-NH₂ |
| TE14015 | 27 | H-Arg-Arg-Nal-Cys-Tyr-Cit-Lys-DGlu-Pro-DGlu-Arg-Cit-Cys-Arg-NH₂ |
| TE14016 | 28 | H-Arg-Arg-Nal-Cys-Tyr-Cit-Lys-DGlu-Pro-Tyr-Arg-DGlu-Cys-Arg-NH₂ |
| AcTE14014 | 29 | Ac-DGlu-Arg-Nal-Cys-Tyr-Cit-Lys-DGlu-Pro-Tyr-Arg-Cit-Cys-Arg-NH₂ |
| AcTE14015 | 30 | Ac-Arg-Arg-Nal-Cys-Tyr-Cit-Lys-DGlu-Pro-DGlu-Arg-Cit-Cys-Arg-NH₂ |
| AcTE14016 | 31 | Ac-Arg-Arg-Nal-Cys-Tyr-Cit-Lys-DGlu-Pro-Tyr-Arg-DGlu-Cys-Arg-NH₂ |
| TF1: AcTE14011 | 32 | Ac-Arg-Arg-Nal-Cys-Tyr-Cit-Lys-DGlu-Pro-Tyr-Arg-Cit-Cys-Arg-NH₂ |
| TF2: guanyl-TE14011 | 33 | guanyl-Arg-Arg-Nal-Cys-Tyr-Cit-Lys-DGlu-Pro-Tyr-Arg-Cit-Cys-Arg-NH₂ |
| TF3: TMguanyl-TE14011 | 34 | TMguanyl-Arg-Arg-Nal-Cys-Tyr-Cit-Lys-DGlu-Pro-Tyr-Arg-Cit-Cys-Arg-NH₂ |
| TF4: TMguanyl-TE14011 (2-14) | 35 | TMguanyl-Arg-Nal-Cys-Tyr-Cit-Lys-DGlu-Pro-Tyr-Arg-Cit-Cys-Arg-NH₂ |
| TF5: 4F-benzoyl-TE14011 | 36 | 4F-benzoyl-Arg-Arg-Nal-Cys-Tyr-Cit-Lys-DGlu-Pro-Tyr-Arg-Cit-Cys-Arg-NH₂ |
| TF6: 2F-benzoyl-TE14011 | 37 | 2F-benzoyl-Arg-Arg-Nal-Cys-Tyr-Cit-Lys-DGlu-Pro-Tyr-Arg-Cit-Cys-Arg-NH₂ |
| TF7: APA-TE14011 (2-14) | 38 | APA-Arg-Nal-Cys-Tyr-Cit-Lys-DGlu-Pro-Tyr-Arg-Cit-Cys-Arg-NH₂ |
| TF8: desamino-R-TE14011 (2-14) | 39 | desamino-R-Arg-Nal-Cys-Tyr-Cit-Lys-DGlu-Pro-Tyr-Arg-Cit-Cys-Arg-NH₂ |
| TF9: guanyl-TE14011 (2-14) | 40 | Guanyl-Arg-Nal-Cys-Tyr-Cit-Lys-DGlu-Pro-Tyr-Arg-Cit-Cys-Arg-NH₂ |
| TF10: succinyl-TE14011 (2-14) | 41 | succinyl-Arg-Nal-Cys-Tyr-Cit-Lys-DGlu-Pro-Tyr-Arg-Cit-Cys-Arg-NH₂ |
| TF11: glutaryl-TE14011 (2-14) | 42 | glutaryl-Arg-Nal-Cys-Tyr-Cit-Lys-DGlu-Pro-Tyr-Arg-Cit-Cys-Arg-NH₂ |
| TF12: deaminoTMG-APA-TE14011 (2-14) | 43 | deaminoTMG-APA-Arg-Nal-Cys-Tyr-Cit-Lys-DGlu-Pro-Tyr-Arg-Cit-Cys-Arg-NH₂ |
| TF15: H-Arg-CH2NH-RTE14011 (2-14) | 44 | R-CH2-Arg-Nal-Cys-Tyr-Cit-Lys-DGlu-Pro-Tyr-Arg-Cit-Cys-Arg-NH₂ |
| TF17: TE14011 (2-14) | 45 | H-Arg-Nal-Cys-Tyr-Cit-Lys-DGlu-Pro-Tyr-Arg-Cit-Cys-Arg-NH₂ |
| TF18: TMguanyl-TC14012 | 46 | TMguanyl-Arg-Arg-Nal-Cys-Tyr-Cit-Lys-DCit-Pro-Tyr-Arg-Cit-Cys-Arg-NH₂ |
| TF19: ACA-TC14012 | 47 | ACA-Arg-Arg-Nal-Cys-Tyr-Cit-Lys-DCit-Pro-Tyr-Arg-Cit-Cys-Arg-NH₂ |
| TF20: ACA-T140 | 48 | ACA-Arg-Arg-Nal-Cys-Tyr-Arg-Lys-DLys-Pro-Tyr-Arg-Cit-Cys-Arg-OH |
| TZ14011 | 49 | H-Arg-Arg-Nal-Cys-Tyr-Cit-Arg-DLys-Pro-Tyr-Arg-Cit-Cys-Arg-NH₂ |
| AcTZ14011 | 50 | Ac-Arg-Arg-Nal-Cys-Tyr-Cit-Arg-DLys-Pro-Tyr-Arg-Cit-Cys-Arg-NH₂ |
| AcTN14003 | 51 | Ac-Arg-Arg-Nal-Cys-Tyr-Cit-Lys-DLys-Pro-Tyr-Arg-Cit-Cys-Arg-NH₂ |
| AcTN14005 | 52 | Ac-Arg-Arg-Nal-Cys-Tyr-Arg-Lys-DCit-Pro-Tyr-Arg-Cit-Cys-Arg-NH₂ |
| 4F-benzoyl-TN14011-Me | 53 | 4F-benzoyl-Arg-Arg-Nal-Cys-Tyr-Cit-Lys-DGlu-Pro-Tyr-Arg-Cit-Cys-Arg-NHMe |
| 4F-benzoyl-TN14011-Et | 54 | 4F-benzoyl-Arg-Arg-Nal-Cys-Tyr-Cit-Lys-DGlu-Pro-Tyr-Arg-Cit-Cys-Arg-NHEt |
| 4F-benzoyl-TN14011-iPr | 55 | 4F-benzoyl-Arg-Arg-Nal-Cys-Tyr-Cit-Lys-DGlu-Pro-Tyr-Arg-Cit-Cys-Arg-NHiPr |
| 4F-benzoyl-TN14011-tyramine | 56 | 4F-benzoyl-Arg-Arg-Nal-Cys-Tyr-Cit-Lys-DGlu-Pro-Tyr-Arg-Cit-Cys-Arg-tyramine |
| TA14001 | 57 | H-Ala-Arg-Nal-Cys-Tyr-Arg-Lys-DLys-Pro-Tyr-Arg-Cit-Cys-Arg-OH |
| TA14005 | 58 | H-Arg-Arg-Nal-Cys-Tyr-Ala-Lys-DLys-Pro-Tyr-Arg-Cit-Cys-Arg-OH |
| TA14006 | 59 | H-Arg-Arg-Nal-Cys-Tyr-Arg-Ala-DLys-Pro-Tyr-Arg-Cit-Cys-Arg-OH |
| TA14007 | 60 | H-Arg-Arg-Nal-Cys-Tyr-Arg-Lys-DAla-Pro-Tyr-Arg-Cit-Cys-Arg-OH |
| TA14008 | 61 | H-Arg-Arg-Nal-Cys-Tyr-Arg-Lys-DLys-Ala-Tyr-Arg-Cit-Cys-Arg-OH |
| TA14009 | 62 | H-Arg-Arg-Nal-Cys-Tyr-Arg-Lys-DLys-Pro-Ala-Arg-Cit-Cys-Arg-OH |
| TA14010 | 63 | H-Arg-Arg-Nal-Cys-Tyr-Arg-Lys-DLys-Pro-Tyr-Ala-Cit-Cys-Arg-OH |
| TC14001 | 64 | H-Cit-Arg-Nal-Cys-Tyr-Arg-Lys-DLys-Pro-Tyr-Arg-Cit-Cys-Arg-OH |
| TC14003 | 65 | H-Arg-Arg-Nal-Cys-Tyr-Cit-Lys-DLys-Pro-Tyr-Arg-Cit-Cys-Arg-OH |
| TN14003 | 66 | H-Arg-Arg-Nal-Cys-Tyr-Cit-Lys-DLys-Pro-Tyr-Arg-Cit-Cys-Arg-NH₂ |
| TC14004 | 67 | H-Arg-Arg-Nal-Cys-Tyr-Arg-Cit-DLys-Pro-Tyr-Arg-Cit-Cys-Arg-OH |
| TC14012 | 68 | H-Arg-Arg-Nal-Cys-Tyr-Cit-Lys-DCit-Pro-Tyr-Arg-Cit-Cys-Arg-NH₂ |
| T-140 | 69 | H-Arg-Arg-Nal-Cys-Tyr-Arg-Lys-DLys-Pro-Tyr-Arg-Cit-Cys-Arg-OH |
| TC14011 | 70 | H-Arg-Arg-Nal-Cys-Tyr-Cit-Lys-DCit-Pro-Tyr-Arg-Cit-Cys-Arg-OH |
| TC14005 | 71 | H-Arg-Arg-Nal-Cys-Tyr-Arg-Lys-DCit-Pro-Tyr-Arg-Cit-Cys-Arg-OH |
| TC14018 | 72 | H-Cit-Arg-Nal-Cys-Tyr-Arg-Lys-DCit-Pro-Tyr-Arg-Cit-Cys-Arg-NH₂ |

According to a specific instance, in each one of SEQ ID NOS:1-72, two cysteine residues are coupled in a disulfide bond.

In another instance, the analog or derivative has an amino acid sequence as set forth in SEQ ID NO:65 (H-Arg-Arg-Nal-Cys-Tyr-Cit-Lys-DLys-Pro-Tyr-Arg-Cit-Cys-Arg-OH; TC14003).

In another instance, the peptide used in the compositions and methods of the invention consists essentially of an amino acid sequence as set forth in SEQ ID NO:1. In another instance, the peptide used in the compositions and methods of the invention comprises an amino acid sequence as set forth in SEQ ID NO:1. In another instance, the peptide is at least 60%, at least 70% or at least 80% homologous to SEQ ID NO:1. In another embodiment, the peptide is at least 90% homologous to SEQ ID NO:1. In another instance, the peptide is at least about 95% homologous to SEQ ID NO:1.

In various other instances, the peptide is selected from SEQ ID NOS:1-72. In another instance, the peptide has an amino acid sequence as set forth in any one of SEQ ID NOS: 1-4, 10, 46, 47, 51-56, 65, 66, 68, 70 and 71. In another instance, the peptide has an amino acid sequence as set forth in any one of SEQ ID NOS: 4, 10, 46, 47, 68 and 70. In another instance, the peptide has an amino acid sequence as set forth in any one of SEQ ID NOS:1, 2, 51, 65 and 66. In another instance, the peptide has an amino acid sequence as set forth in any one of SEQ ID NOS:53-56.

In an instance, the peptide has an amino acid sequence as set forth in SEQ ID NO:1. In another instance, the peptide has an amino acid sequence as set forth in SEQ ID NO:2. In another instance, the peptide has an amino acid sequence as set forth in SEQ ID NO:51. In another instance, the peptide has an amino acid sequence as set forth in SEQ ID NO:66.

Other CXCR4 peptide inhibitors (antagonists) include but are not limited to LY2510924 (by Lilly Oncology), CTCE-9908 (Huang et al. 2009 Journal of Surgical Research 155:231-236), Fc131 analogs and nanobodies as specified in the citations below: Tan NC, Yu P, Kwon Y-U, Kodadek T. High-throughput evaluation of relative cell permeability between peptoids and peptides. Bioorg Med Chem. 2008;16:5853-61.

Kwon Y-U, Kodadek T. Quantitative evaluation of the relative cell permeability of peptoids and peptides. J Am Chem Soc. 2007;129:1508.

Miller S, Simon R, Ng S, Zuckermann R, Kerr J, Moos W. Comparison of the proteolytic susceptibilities of homologous L-amino acid, D-amino acid, and N-substituted glycine peptide and peptoid oligomers. Drug Dev Res. 1995;35:20-32.

Yoshikawa Y, Kobayashi K, Oishi S, Fujii N, Furuya T. Molecular modeling study of cyclic pentapeptide CXCR4 antagonists: new insight into CXCR4-FC131 interactions. Bioorg Med Chem Lett. 2012;22:2146-50.

Jaähnichen S, Blanchetot C, Maussang D, Gonzalez-Pajuelo M, Chow KY, Bosch L, De Vrieze S, Serruys B, Ulrichts H, Vandevelde W. CXCR4 nanobodies (VHH-based single variable domains) potently inhibit chemotaxis and HIV-1 replication and mobilize stem cells. Proc Natl Acad Sci USA. 2010;107:20565-70.

Without being bound by theory it is suggested that peptide of the present invention induces growth arrest and/or death of myeloid leukemia cells.

As used herein, the phrase "chemotherapeutic agent" refers to any chemical agent with therapeutic usefulness in the treatment of cancer. Chemotherapeutic agents as used herein encompass both chemical and biological agents. These agents function to inhibit a cellular activity upon which the cancer cell depends for continued survival. Categories of chemotherapeutic agents include alkylating/alkaloid agents, antimetabolites, hormones or hormone analogs, and miscellaneous antineoplastic drugs. Most if not all of these drugs are directly toxic to cancer cells and do not require immune stimulation. Suitable chemotherapeutic agents are described, for example, in Slapak and Kufe, Principles of Cancer Therapy, Chapter 86 in Harrison's Principles of Internal medicine, 14th edition; Perry et al., Chemotherapeutic, Ch 17 in Abeloff, Clinical Oncology 2nd ed., 2000 ChrchillLivingstone, Inc.; Baltzer L. and Berkery R. (eds): Oncology Pocket Guide to Chemotherapeutic, 2nd ed. St. Luois, mosby-Year Book, 1995; Fischer D. S., Knobf M. F., Durivage H.J. (eds): The Cancer Chemotherapeutic Handbook, 4th ed. St. Luois, Mosby-Year Handbook.

In some instances the chemotherapeutic agent of the present disclosure is cytarabine (cytosine arabinoside, Ara-C, Cytosar-U), carboplatin, carmustine, chlorambucil, dacarbazine, ifosfamide, lomustine, mechlorethamine, procarbazine, pentostatin, (2'deoxycoformycin), etoposide, teniposide, topotecan, vinblastine, vincristine, paclitaxel, dexamethasone, methylprednisolone, prednisone, all-trans retinoic acid, arsenic trioxide, interferon-alpha, rituximab (Rituxan®), gemtuzumab ozogamicin, imatinib mesylate, Cytosar-U), melphalan, busulfan (Myleran®), thiotepa, bleomycin, platinum (cisplatin), cyclophosphamide, Cytoxan®)., daunorubicin, doxorubicin, idarubicin, mitoxantrone, 5-azacytidine, cladribine, fludarabine, hydroxyurea, 6-mercaptopurine, methotrexate, 6-thioguanine, or any combination thereof.

In the embodiment the chemotherapeutic agent is cytarabine.

As used herein "Cytarabine" also known as "cytosine arabinoside" is a chemotherapy agent which interferes with DNA synthesis.

Brand names include, but are not limited to, Cytostar-U, Tarabine PFS (Pfizer), Depocyt (longer lasting liposomal formulation) and Ara-C (Arabinofuranosyl Cytidine).

The CXCR4-antagonistic peptide and the chemotherapeutic agent of the present invention are used for treating myeloid leukemia. In one embodiment the myeloid leukemia is acute myeloid leukemia (AML). Methods of diagnosing and monitoring acute myeloid leukemia are described, for example, in Cheson et al., J Clin Oncol 21(24):4642-4649, 2003.

As used herein, the term "treating" refers to inhibiting, preventing or arresting the development of a pathology (disease, disorder or condition i.e., myeloid leukemia) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

As used herein, the term "preventing" refers to keeping a disease, disorder or condition from occurring in a subject who may be at risk for the disease, but has not yet been diagnosed as having the disease.

As used herein, the term "subject" includes mammals, preferably human beings at any age which suffer from the pathology, myeloid leukemia e.g., acute myeloid leukemia or chronic myeloid leukemia.

The CXCR4-antagonistic peptide and the chemotherapeutic agent of the invention can be administered concomitantly or sequentially.

In some instances the CXCR4-antagonistic peptide is administered at least 1 hour, at least 2 hours, at least 4 hours, at least 8 hours, at least 12 hours, at least 1 day, at least 2 days, at least 3 days, at least 4 days, at least 5 days, at least 6 days, at least 1 week, or at least 1 month prior to the administration of the chemotherapeutic agent.

According to some instances, the CXCR4-antagonistic peptide is administered between 1 to 24 hours prior to the administration of the chemotherapeutic agent. According to some instances, the CXCR4-antagonistic peptide is administered between 1 to 8 hours prior to the administration of the chemotherapeutic agent.

The CXCR4-antagonistic peptide and the chemotherapeutic agent of the invention can each be administered to the subject as active ingredients *per se,* or in a pharmaceutical composition where each of the active ingredients is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the peptides accountable for the biological effect. Optionally, a plurality of active ingredient may be included in the formulation such as chemotherapeutic, radiation agents and the like, as further described hereinbelow.

Hereinafter, the phrases "physiologically acceptable carrier" and "pharmaceutically acceptable carrier", which may be used interchangeably, refer to a carrier or a diluent that does not cause significant irritation to an organism and does not abrogate the biological activity and properties of the administered compound.

Herein, the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils, and polyethylene glycols.

Techniques for formulation and administration of drugs may be found in the latest edition of "Remington's Pharmaceutical Sciences", Mack Publishing Co., Easton, PA (Remington: The Science and Practice of Pharmacy, Gennaro, A., Lippincott, Williams & Wilkins, Philadelphia, Pa., 20th ed, 2000).

Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

In one instance, the peptide of the invention or the pharmaceutical composition comprising same is administered subcutaneously.

In another instance, the chemotherapeutic agent of the invention or the pharmaceutical composition comprising same is administered intravenously.

For injection, the active ingredients of the pharmaceutical composition may be formulated in aqueous solutions (e.g., WFI), preferably in physiologically compatible buffers such as Hank's solution, Ringer's solution, or physiological salt buffer.

Pharmaceutical compositions for potential administration include aqueous solutions of the active preparation in water-soluble form. Additionally, suspensions of the active ingredients may be prepared as appropriate oily or water-based injection suspensions. Suitable lipophilic solvents or vehicles include fatty oils such as sesame oil, or synthetic fatty acid esters such as ethyl oleate, triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the active ingredients, to allow for the preparation of highly concentrated solutions.

Alternatively, the active ingredient may be in powder form for constitution with a suitable vehicle, e.g., a sterile, pyrogen-free, water-based solution, before use.

Alternative instances include depots providing sustained release or prolonged duration of activity of the active ingredient in the subject, as are well known in the art.

Pharmaceutical compositions suitable for use in the context of the present invention include compositions wherein the active ingredients are contained in an amount effective to achieve the intended purpose. Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals (see the Examples section which follows, and Sekido et al. 2002 Cancer Genet Cytogenet 137(1):33-42). The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

In some instances the daily dose of the CXCR4-antagonistic peptide of the invention or the pharmaceutical composition comprising same is ranging between 0.1 to 10 mg/kg of body weight, between 0.1 to 2 mg/kg of body weight, between 0.1 to 1 mg/kg of body weight, between 0.3 to 10 mg/kg of body weight, between 0.3 to 2 mg/kg of body weight, between 0.3 to 1 mg/kg of body weight or between 0.3 to 0.9 mg/kg of body weight.

In some instances the daily dose the chemotherapeutic agent of the invention or the pharmaceutical composition comprising same is ranging between 1 to 10 g per square meter of body area, between 1.5 to 5 g per square meter of body area or between 2 to 4 g per square meter of body area.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary depending on a number of clinical factors, such as blood counts (e.g., red or white blood cell levels, hemoglobin level, etc.) the subject sensitivity to the peptide and/or the chemotherapeutic agent. The desired dose can be administered at one time or divided into sub-doses, e.g., 2-4 sub-doses and administered over a period of time, e.g., at appropriate intervals through the day or other appropriate schedule. Such sub-doses can be administered as unit dosage forms.

In some instances the CXCR4-antagonistic peptide of the invention is administered for a period of at least 1 day, at least 2 days, at least 3 days, at least 4 days, at least 5 days, at least 6 days, at least 1 week, at least 2 weeks, at least 3 weeks, at least 1 month, or at least 2 months prior to administering of the chemotherapeutic agent.

The active ingredients described herein can be packaged in an article of manufacture which comprises at least two separate containers. One container packaging the CXCR-4 peptide antagonist (e.g., peptide set forth in SEQ ID NO: 1) and another container which packages the chemotherapy (e.g., ara-C). The article of manufacture may comprise a label and/or instructions for the treatment of myeloid leukemia (e.g., AML).

Alternatively or additionally, the CXCR4 inhibitor (e.g., SEQ ID NO: 1) and chemotherapy (cytarabine) can be formulated in a pharmaceutical composition as described above as a co-formulation.

Thus, compositions (CXCR4 antagonist, chemotherapy or a combination of same) and/or articles of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container (e.g., lyophilized vial), and labeled for treatment of an indicated condition, as is further detailed above.

As used herein the term "about" refers to ± 10 %.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Various embodiments and aspects of the present invention as delineated hereinabove and as claimed in the claims section below find experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., Ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (Eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., Ed. (1994); "Culture of Animal Cells - A Manual of Basic Technique" by Freshney, Wiley-Liss, N. Y. (1994), Third Edition; "Current Protocols in Immunology" Volumes I-III Coligan J. E., Ed. (1994); Stites et al. (Eds.), "Basic and Clinical Immunology" (8th Edition), Appleton & Lange, Norwalk, CT (1994); Mishell and Shiigi (Eds.), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., Ed. (1984); "Nucleic Acid Hybridization" Hames, B. D., and Higgins S. J., Eds. (1985); "Transcription and Translation" Hames, B. D., and Higgins S. J., Eds. (1984); "Animal Cell Culture" Freshney, R. I., Ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### 4F-benzoyl-TN14003 Alleviates Cytarabine-Induced Toxicity in Mice Materials and Methods

### Reagents

### 4F-benzoyl-TN14003

Lyophilized 4F-benzoyl-TN14003 was manufactured by MSD N.V. The compound was dissolved with water for injection (WFI) at a final stock concentration of 25 mg/ml and stored at -20°C until use. Before injection to the mice, 4F-benzoyl-TN14003 was thawed and diluted with PBS to final concentration of 5 mg/ml. Actual dosing solutions were prepared by diluting the 5 mg/ml stock solution (total dose including salt) in PBS just before the injection. 4F-benzoyl-TN14003 was administered at a constant dose volume of 200 µL.

### Cytarabine

Cytarabine (Cytosine arabinoside; ARA-C) was purchased from Hadassah cytotoxica pharmacy (Israel). ARA-C was provided from Hadassah cytotoxica pharmacy at concentration of 100 mg/ml. Actual dose solution (200 mg/Kg) was prepared by diluting the 100 mg/ml stock solution in PBS.

### Animals

Normal C57BL/6 female mice, 9 - 10 week old, about 20 gram in weight, were used. The animals were kept in groups of a maximum of 10 animals in polysulphone cages (425x266x185mm), fitted with solid bottoms and filled with wood shavings as bedding material. The animals were provided *ad libitum* a commercial rodent diet (Harlan Teklad™ Ra/Mouse Diet) and allowed free access to autoclaved water, supplied to each cage via polysulphone bottles with stainless steel sipper-tubes. From the first day of ARA-C dosing wet food was placed at the bottom of the cage. Ten animals were randomly allocated per treatment group.

### Toxicity assay

The treatments groups were as followed (10 animals per treatment group):
- Group A: untreated control.
- Group B: animals administered with 4FB-TN14003 at a dose of 2.4, 4.8, 9.6 or 12 mg/Kg daily from day 1 to day 7.
- Group C: animals administered with ARA-C at a dose of 200 mg/Kg daily from day 3 to 7.
- Group D: animals administered with 4FB-TN14003 as in group B (at a dose of 2.4, 4.8, 9.6 or 12 mg/Kg daily from day 1 to day 7) and also with ARA-C as in group C (at a dose of 200 mg/Kg daily from day 3 to 7).

4FB-TN14003 was injected SC at a constant dose volume of 200 µL/mouse (based on the latest determined body weight- average of 20 gr), once daily for 7 consecutive days (days 1-7). Control mice were injected with the vehicle (PBS) only under the same regimen.

ARA-C was diluted in PBS and injected SC at a constant dose of 200 mg/kg and at constant volume of 200 µL/mouse, once daily for 5 consecutive days (days 3-7). In the combination groups (groups 7-10) ARA-C was injected 4 hours following 4FB-TN14003 injection.

Blood samples were collected on day 12 of the experiment. The mice in each group were subjected to terminal bleeding from the orbital sinus. Blood samples (ca. 400-500 µl) were dispensed into special serum gel separation tubes (BD Microgard™), centrifuged at 13,000 rpm for 8 minutes at RT and saved the supernatant sera. Sera samples (at least 220 µl) were kept at 2-8°C to complete blood counts (CBC). CBC was done using a Sysmex KX-21 automatic multi-parameter blood cell counter (Sysmeex, USA) essentially as described by Nervi et al. (Blood 113(24):6206-14, 2009).

### Results

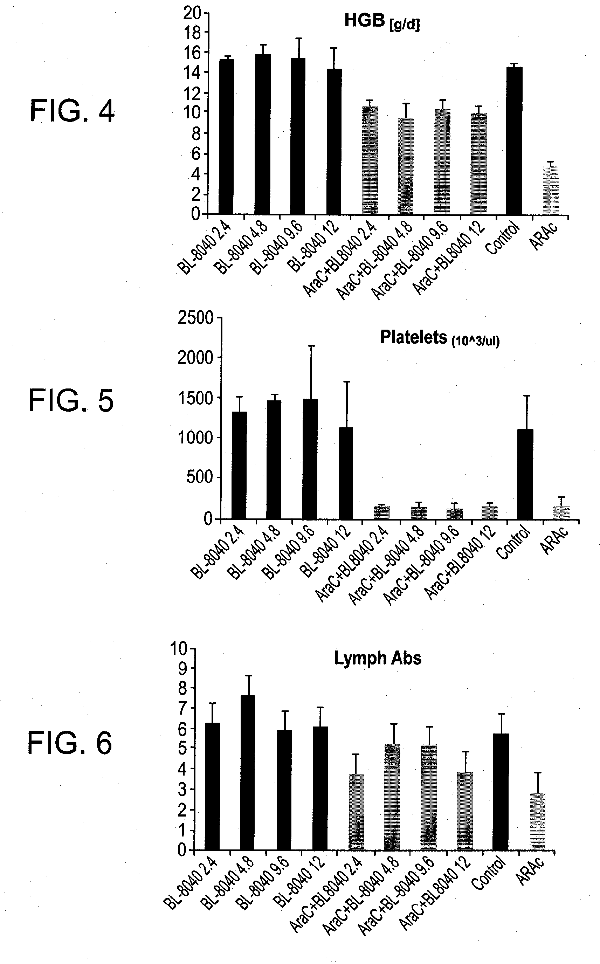

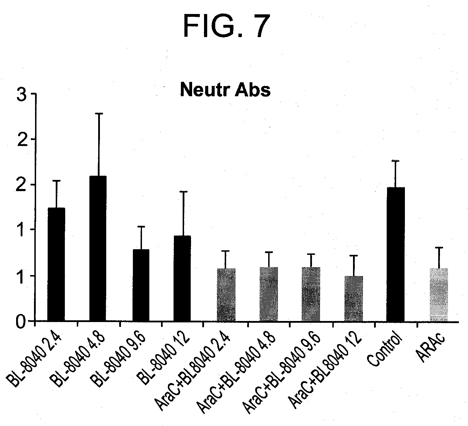

Treatment with ARA-C alone, when compared with the untreated control, resulted, as expected, in a drastic reduction in white blood-cells (Figure 1), red blood-cells (Figure 2), hematocrit (Figure 3), hemoglobin (Figure 4), platelets (Figure 5), lymphocyte Abs (Figure 6) and neutrophil Abs (Figure 7).

Treatment with ARA-C alone, or in combination with 4F-benzoyl-TN14003, resulted in equally reduced levels of platelets (Figure 5) and neutrophil Abs (Figure 7).

Treatment with 4F-benzoyl-TN14003 alone, when compared with the untreated control, did not cause a reduction in white blood-cells (Figure 1), red blood-cells (Figure 2), hematocrit (Figure 3), hemoglobin (Figure 4), platelets (Figure 5) and lymphocyte Abs (Figure 6).

Most surprisingly, treatment with 4F-benzoyl-TN14003 combined with ARA-C resulted in substantial *increase* in red blood-cells, hematocrit and hemoglobin, when compared to mice treated with ARA-C only (Figures 2, 3 and 4, respectively).

These results indicate that the CXCR4-antagonistic peptide is capable of alleviating some of the non-target toxic injury caused by the chemotherapeutic agent.

## Claims

1. A therapeutically effective amount of a CXCR4-antagonistic peptide and a therapeutically effective amount of a chemotherapeutic agent for use in the treatment of myeloid leukemia in a subject in need thereof, wherein said CXCR4-antagonistic peptide is as set forth in SEQ ID NO: 1 and said chemotherapeutic agent is cytarabine.

2. The CXCR-4 antagonistic peptide and the chemotherapeutic agent for use according to claim 1, wherein the myeloid leukemia is acute myeloid leukemia.

3. The CXCR-4 antagonistic peptide and the chemotherapeutic agent fore use according to claim 1 or 2, wherein said CXCR4-antagonistic peptide is administered to said subject in a daily amount between 0.1 to 10 mg per kg of body weight or wherein cytarabine is administered to said subject in a daily amount between 1 to 10 g per square meter of body area.

## Patentansprüche

1. Therapeutisch wirksame Menge an CXCR4-antagonistischem Peptid und therapeutisch wirksame Menge an einem chemotherapeutischen Mittel zur Verwendung bei der Behandlung von myeloischer Leukämie in einem dies benötigenden Subjekt, wobei das CXCR4-antagonistische Peptid wie in SEQ ID NO: 1 dargelegt ist und das chemotherapeutische Mittel Cytarabin ist.

2. CXCR4-antagonistisches Peptid und chemotherapeutisches Mittel zur Verwendung nach Anspruch 1, wobei die myeloische Leukämie akute myeloische Leukämie ist.

3. CXCR4-antagonistisches Peptid und chemotherapeutisches Mittel zur Verwendung nach Anspruch 1 oder 2, wobei das CXCR4-antagonistische Peptid an das Subjekt in einer täglichen Menge zwischen 0,1 und 10 mg pro kg Körpergewicht verabreicht wird oder wobei Cytarabin an das Subjekt in einer täglichen Menge zwischen 1 und 10 g pro Quadratmeter Körperfläche verabreicht wird.

## Revendications

1. Quantité thérapeutiquement efficace d'un peptide antagoniste de CXCR4 et quantité thérapeutiquement efficace d'un agent chimiothérapeutique pour utilisation dans le traitement de la leucémie myéloïde chez un sujet en ayant besoin, ledit peptide antagoniste de CXCR4 étant tel que présenté dans SEQ ID NO : 1 et ledit agent chimiothérapeutique étant la cytarabine.

2. Peptide antagoniste de CXCR4 et agent chimiothérapeutique pour utilisation selon la revendication 1, la leucémie myéloïde étant une leucémie myéloïde aiguë.

3. Peptide antagoniste de CXCR4 et agent chimiothérapeutique pour utilisation selon la revendication 1 ou 2, ledit peptide antagoniste de CXCR4 étant administré audit sujet à une dose quotidienne comprise entre 0,1 et 10 mg par kilo de poids corporel ou la cytarabine étant administrée audit sujet à une dose quotidienne comprise entre 1 et 10 g par mètre carré de surface corporelle.
